# EUROPÄISCHE PATENTSCHRIFT

(11) **EP 2 853 219 B1**
(45) Veröffentlichungstag und Bekanntmachung des Hinweises auf die Patenterteilung: **06.06.2018**
(21) Anmeldenummer: 13184185.0
(22) Anmeldetag: 12.09.2013
(51) Int. Cl.: A61B 18/14

(54) **Instrument zur Gefäßversiegelung**
Vessel sealing instrument
Instrument permettant de sceller un vaisseau

(30) Priorität: 10.09.2013 EP 13183641
(43) Veröffentlichungstag der Anmeldung: 01.04.2015
(60) Teilanmeldung: 18157385.8
(73) Patentinhaber: Erbe Elektromedizin GmbH, 72072 Tübingen (DE)
(72) Erfinder: Amann, Marcus, 72070 Tübingen (DE); Schäller, Daniel, 72074 Tübingen (DE); Mayer, Volker, 72072 Tübingen (DE); Heim, Martina, 72124 Pliezhausen (DE)
(74) Vertreter: Rüger, Barthelt & Abel

(56) Entgegenhaltungen:
- EP-A1- 2 377 480
- WO-A1-02/080785
- US-A1- 2011 073 246
- US-A1- 2013 185 922

## Beschreibung

Die Erfindung betrifft ein chirurgisches Instrument zur Gewebekoagulation, insbesondere zur Gefäßversiegelung. Solche Instrumente können zum Beispiel zum Verschließen, Verschweißen und Koagulieren von Gewebe, beispielsweise Blutgefäßen, eingesetzt werden. Es kann vorgesehen sein, das Gewebe nach dem Koagulieren auch zu durchtrennen.

Im Folgenden beschreibt der Begriff "distal" stets den vom Anwender entfernten Teil des Instruments oder Bauteils, der Begriff "proximal" beschreibt stets den zum Anwender hin gerichteten näher liegenden Teil des Instruments oder Bauteils.

Instrumente der genannten Bauart sind grundsätzlich bekannt. Zum Beispiel offenbart die EP 2371316 A1 ein solches Instrument mit einem länglichen Schaft, der an seinem proximalen Ende an einem Gehäuse gehalten ist, von dem er sich weg erstreckt. Ungefähr rechtwinklig zu dem Schaft sind an dem Gehäuse ein Handgriff und ein Betätigungshebel ausgebildet. An dem distalen Ende des Schafts ist ein zangenartiges Werkzeug mit zwei Branchen angeordnet, die auch als Maulteile bezeichnet werden. Während eines der Maulteile fest angeordnet ist, ist das andere auf das feste Maulteil hin und von diesem weg beweglich. Um es zu bewegen, ist es über Zugmittel und ein Getriebe mit dem Betätigungshebel verbunden.

Die Maulteile sind während des Einsatzes bestrombar, um ein zwischen den Maulteilen gefasstes biologisches Gewebe, beispielsweise ein Blutgefäß, mittels Stromdurchflusses zu erwärmen. Außerdem kann das Werkzeug ein Messer umfassen, um ein zwischen den Branchen gefasstes und fusioniertes Gefäß durchtrennen zu können.

Aus der EP 2 377 480 A1 ist ein ähnliches Instrument bekannt, dessen Branchen Edelstahlelektroden mit Kupferplattierung aufweisen. Die Elektroden sind in äußeren Isoliergehäuse erfasst, die durch Kunststoffspritzguss hergestellt sind.

Weiter offenbart die US 2011/0073246 A1 ein ähnliches Instrument mit Kunststoffbranchen, in die Elektrodeninlays eingeformt sind. Diese weisen einen abgewinkelten Rand auf, in dem Vertiefungen angebracht sind, die den Kunststofffluss während des Spritzgussverfahrens steuern sollen.

Solche Instrumente sind sowohl als sterilisierbare Instrumente als auch als Einweginstrumente in Gebrauch. Bei Einweginstrumenten kommt es auf einen einfachen und kostengünstigen Aufbau an, wobei jedoch keine Kompromisse hinsichtlich der Funktionalität erwünscht sind. Bei Mehrweginstrumenten kommt es auf leichte Reinigbarkeit und Sterilisierbarkeit und Robustheit gegenüber hohen Temperaturen oder sonstigen Sterilisationseinwirkungen an. Auch hier sind Kompromisse hinsichtlich der Funktionalität nicht erwünscht bzw. akzeptabel.

Davon ausgehend ist es Aufgabe der Erfindung, ein Instrument mit einfachem und robustem Aufbau sowie hoher Funktionalität zu schaffen.

Diese Aufgabe wird mit dem Instrument nach Anspruch 1 gelöst:
Das erfindungsgemäße Instrument weist ein Werkzeug mit wenigstens einer beweglichen Branche auf, die einen Branchenträger und eine Elektrodeneinheit umfasst. Die Elektrodeneinheit besteht aus einem vorzugsweise als Stanzbiegeteil hergestellten Blechteil, das teilweise in einem Kunststoffkörper eingelassen und in diesem verankert ist. Dazu weist das Blechteil an zwei langen, einander gegenüber liegenden Rändern Streifenabschnitte auf, die gegenüber den Gewebekontaktflächen abgewinkelt sind. Diese Streifenabschnitte erstrecken sich in den Kunststoffkörper hinein, der somit den Raum zwischen den Streifenabschnitten ausfüllt, die Streifenabschnitte umhüllt und vorzugsweise die Gewebekontaktflächen frei lässt.

Die Streifenabschnitte sind mit Öffnungen versehen, durch die sich der Kunststoffkörper hindurch erstreckt und so einen Formschluss mit dem Blechteil bildet. Der Kunststoffkörper kann durch Umspritzen des Blechteils bzw. seiner abgewinkelten Streifenabschnitte hergestellt sein. Das mit dem Kunststoff umspritzte Blechteil bildet die Elektrodeneinheit.

Vorzugsweise sind die Öffnungen in den Streifenabschnitten großflächig ausgebildet. Sie können durch Rundlöcher, eckige Löcher oder, wie es bevorzugt wird, Schlitze gebildet sein. Diese Bauform ermöglicht eine einfache und rationelle Herstellung. Dies kommt der Verwendung des chirurgischen Instruments als Einweginstrument entgegen.

Das vorgeschlagene Konzept führt beim Einsatz des Instruments zu einer Wärmebelastung des Kunststoffs, die von der Form und der Gestaltung des Blechteils beeinflusst wird. Die sich in den Kunststoff hinein erstreckenden Streifenabschnitte können aufgrund ihrer Öffnungen nur begrenzte Wärmemengen in diesen Bereich in den Kunststoffkörper eintragen. Die in den Streifenabschnitten versehenen Öffnungen unterbrechen und beschränken somit den Wärmefluss von den Gewebekontaktflächen in den Randbereich des Kunststoffkörpers hinein. Dies hat zur Folge, dass Gewebe, welches an dem Kunststoffkörper in diesem Randbereich anliegt weniger ungewünschte thermische Beschädigung erfährt.

Die erfindungsgemäße Bauform der Elektrodeneinheit führt außerdem zu gut reproduzierbaren Koagulationsergebnissen. Die Wärmekapazität des Blechteils und die Wärmeleitfähigkeit des Kunststoffkörpers sind relativ gering. Die Gewebekontaktfläche kann sich somit während des Einsatzes zusammen mit dem biologischen Gewebe hoch dynamisch und ohne große Verzögerung auf die gewünschte Gewebetemperatur erwärmen. Dies kommt der Handhabungssicherheit und einer reduzierten Bearbeitungszeit entgegen. Der Anwender erhält nahezu einheitliche Koagulations- bzw. Versiegelungsergebnisse unabhängig davon, ob er ein noch nicht benutztes kaltes Instrument oder ein Instrument nutzt, mit dem er kurz zuvor bereits eine oder mehrere Koagulationen bzw. Versiegelungen durchgeführt hat und dessen Gewebekontaktflächen eine erhöhte Anfangstemperatur haben.

Ein weiterer Vorzug der Einbettung dünner, als Elektroden bzw. Gewebekontaktflächen vorgesehener Blechteile in den Kunststoffkörper liegt in der thermischen und elektrischen Isolation des Blechteils gegen umgebendes Gewebe. Es werden so unerwünschte Koagulationseffekte in der Nähe der Eingriffsstelle minimiert.

Die zu dem erfindungsgemäßen Instrument gehörige Elektrodeneinheit kann sowohl von Branchenträgern aus Kunststoff als auch von Branchenträgern aus Metall getragen werden. Letzteres wird bevorzugt. Dabei ist insbesondere die elektrische Isolation des Blechteils von dem jeweiligen tragenden Branchenträger durch den Kunststoffkörper von Vorteil. Aus Metall bestehende Branchenträger sind elektrisch von den Gewebekontaktflächen entkoppelt sowie thermisch nur gering von den Elektrodeneinheiten beeinflusst und führen somit nicht zu Effekten in dem umgebenden Gewebe. Dies eröffnet dem Anwender auch die Möglichkeit der Gefäßversiegelung unter erschwerten Bedingungen, beispielsweise bei beschränktem Zugang zu dem zu bearbeitenden Gewebe.

Wenn zwischen den Öffnungen, die in den Streifenabschnitten ausgebildet sind, Stege angeordnet sind, deren Breite nicht größer als ihre Länge ist, wird der Wärmefluss von diesen Streifenabschnitten ausgehend in den Kunststoff körper hinein merklich reduziert. Dies gilt in besonderem Maße, wenn die Fläche der Öffnungen größer ist, als die Fläche der zwischen den Öffnungen vorhandenen Stege. Insbesondere wird es als vorteilhaft angesehen, wenn das Verhältnis der Flächen der Öffnungen zu der Fläche der Stege so groß wie möglich ist, und im Bereich von 3 zu 1 bis 20 zu 1, vorzugsweise 10 zu 1 liegt.

Der Kunststoffkörper ist so ausgebildet, dass er seitlich neben der Gewebekontaktfläche einen isolierenden Bereich aufweist. Dieser Bereich kann als Stufe oder andersartig geformte Absetzung beispielsweise in Form einer Schräge ausgebildet sein. Dies führt zu einem ausreichenden Abstand zwischen den Gewebekontaktflächen und umgebendem nicht zu beeinflussenden Gewebe.

Die Gewebekontaktfläche ist vorzugsweise durch einen Längsschlitz unterbrochen. In diesem kann ein längsbewegliches Messer geführt sein. Vorzugsweise ist bei dieser Bauform in dem Kunststoffkörper in fluchtender Ausrichtung zu dem Längsschlitz eine Messerführungsnut vorgesehen. Diese ist vorzugsweise schmaler als der Längsschlitz. Dadurch wird das Messer von dem Blechteil elektrisch isoliert. Auf diese Weise werden sowohl elektrische Kurzschlüsse zwischen den Gewebekontaktflächen der beiden Branchen und dem Messer vermieden.

Das Blechteil kann, wie erwähnt, als Stanzbiegeteil gebildet sein. Es kann auch auf andere Art und Weise, beispielsweise durch Laserbearbeitung, Wasserstrahltechnologie oder chemisch durch Ätzen oder andere Verfahren, hergestellt werden. Vorzugsweise sind in der Gewebekontaktfläche ein oder mehrere Ausnehmungen, beispielsweise in Form von Bohrungen, ausgebildet, durch die sich der Kunststoffkörper erstreckt, um Haltemittel zu bilden. Auf diese Weise kann ein elektrischer Kurzschluss zwischen den beiden Blechteilen beim Schließen der Branchen vermieden und die Fixierung des gefassten Gewebes während des Versiegelungsvorgangs verbessert werden. Die Haltemittel können zu beiden Seiten des Messerschlitzes gegeneinander versetzt oder symmetrisch angeordnet sein. Die Haltemittel können auch bezüglich den gegenüber liegenden Branchen versetzt angeordnet sein, so dass beim Schließen der Branchen die Haltemittel nicht aufeinander treffen, sondern die gegenüberliegende Gewebekontaktfläche berühren können. Die Haltemittel klemmen das während des Versiegelungsvorgangs schrumpfende Gewebe, wodurch die Qualität der Versiegelung erhöht wird. Zudem können diese Haltemittel auch als Abstandshalter zwischen den Gewebekontaktflächen dienen.

Die flächenhafte Ausdehnung der einzelnen Haltemittel hat einen Einfluss auf das Versiegelungsergebnis und auf das Halten des geklemmten Gewebes. Deshalb ist es vorteilhaft, wenn die Flächeninhalte der einzelnen Haltemittel in einem Bereich von 0,5 mm² bis 7 mm² vorzugsweise 1 mm² insbesondere vorzugsweise 0,75 mm² liegen.

Zur Verhinderung des elektrischen Kontakts zwischen den Gewebekontaktflächen der Branchen sind vorzugsweise am Rand der Gewebekontaktflächen der Blechteile, insbesondere am distalen Ende derselben, Abstandshalter angeordnet. Diese können durch Vorsprünge des Kunststoffkörpers gebildet sein, die die Gewebekontaktfläche überragen.

Bei jedem der vorgenannten Instrumente können die zwei Elektrodeneinheiten an Branchenträgern aus Metall befestigt sein. Außerdem können bei jedem der vorgenannten Instrumente die Branchen beweglich gelagert sein. Vorzugsweise stehen die Branchen miteinander in elektrischem Kontakt.

Die beiden Elektrodeneinheiten sind vorzugsweise an beweglich gelagerten Branchenträgern befestigt. Die Branchenträger können miteinander derart in elektrischem Kontakt stehen, so dass sie auf einem einheitlichen elektrischen Potential liegen, welches vorzugsweise floatend und damit nicht elektrisch kontaktiert ist. Auch dies dient dazu, unerwünschte Effekte auf umgebendes Gewebe zu minimieren.

Weitere Einzelheiten vorteilhafter Ausführungsformen der Erfindung sind Gegenstand von Ansprüchen, der Zeichnung oder der Beschreibung. Es zeigen:
Figur 1 das erfindungsgemäße Instrument, in perspektivischer Prinzipdarstellung.
Figur 2 das an dem distalen Ende des Instruments gehaltene Werkzeug mit zwei Branchen, in schematischer Perspektivdarstellung,
Figur 3 ein Sockelteil zur Aufnahme und Lagerung zweier Branchen,
Figur 4 einen Branchenträger zur Aufnahme der Elektrodeneinheit,
Figur 5 die Elektrodeneinheit einer Branche des Werkzeugs nach Figur 2, in perspektivischer Darstellung,
Figur 6 die Elektrodeneinheit nach Figur 5, in perspektivischer Schnittdarstellung,
Figur 6a die Schulter der Elektrodeneinheit nach Figur 6 in vergrößerter, ausschnittsweiser Darstellung,
Figur 7 - 10 ein Blechteil zum Aufbau der Elektrodeneinheit nach Figur 5 und 6, in verschiedenen Ansichten und
Figur 11 und 12 ein Blechteil zum Aufbau der Elektrodeneinheit nach Figur 5 und 6 mit verschiedenen Ausführungsformen der Streifenabschnitte.

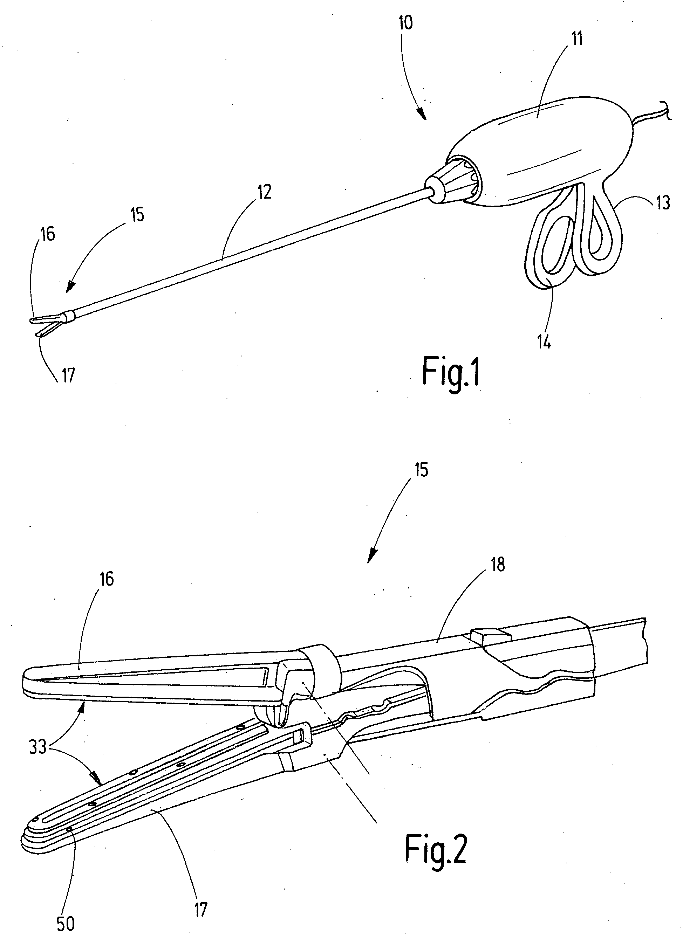

In Figur 1 ist ein chirurgisches Instrument 10 veranschaulicht, das für die offenchirurgische Gefäßversiegelung eingerichtet ist. Das Instrument 10 weist ein Gehäuse 11 auf, von dem sich ein vorzugsweise gerader Schaft 12 weg erstreckt. An dem Gehäuse 11 ist ein Handgriff 13 vorgesehen, in dessen Nachbarschaft ein Bedienhebel 14 schwenkbar gelagert ist. Dieser dient zur Betätigung eines Werkzeugs 15, das an dem distalen Ende des Schafts 12 angebracht ist. Das Instrument 10 kann als Einweginstrument ausgebildet und somit für den einmaligen Gebrauch vorgesehen sein. Das Instrument 10 kann jedoch auch als sterilisierbares und somit wiederverwendbares Instrument ausgebildet sein.

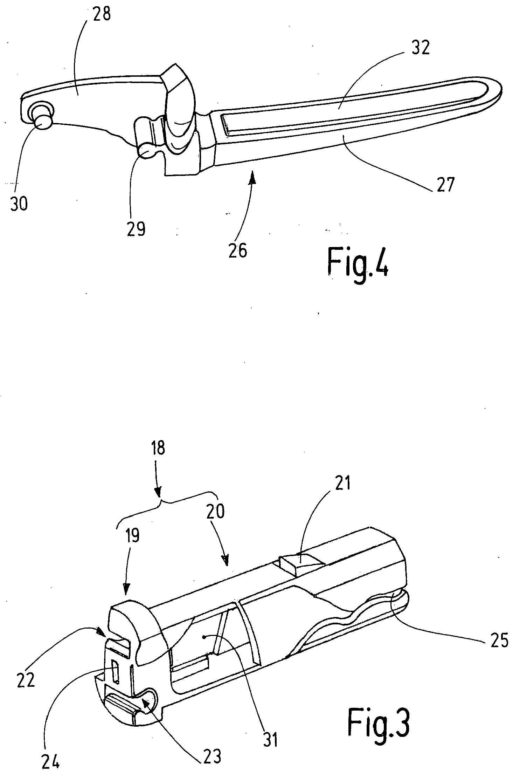

Die Besonderheit des Instruments 10 liegt in der Ausbildung des Werkzeugs 15, das in Figur 2 gesondert veranschaulicht ist. Das Werkzeug 15 weist zwei Branchen 16, 17 auf, von denen wenigstens eine, im vorliegenden Ausführungsbeispiel beide, an einem Sockelteil 18 beweglich gelagert sind. Das Sockelteil 18 kann zum Beispiel aus Kunststoff, Keramik, Verbundmaterial oder auch aus Metall bestehen. In Figur 3 ist das Sockelteil 18 gesondert veranschaulicht. Es weist einen Lagerabschnitt 19 und einen sich davon weg erstreckenden Fortsatz 20 auf, der in das distale Ende des Schafts 12 eingesteckt und mit diesem mittels einer Rastnase 21 verrastet ist. In dem Lagerabschnitt 19 sind zwei zueinander parallele, zu unterschiedlichen Flanken hin offene im Wesentlichen zylindrische Lagerbuchsen 22, 23 ausgebildet, die außerdem zum distalen Ende des Sockelteils 18 hin offen sind. Zwischen den beiden Lagerbuchsen 22, 23 kann ein Schlitz 24 vorgesehen sein, durch den ein Messer zum Durchtrennen koagulierten Gewebes, zum Beispiel versiegelter Gefäße, geschoben werden kann.

Der innen hohl ausgebildete Fortsatz 20 kann außen an seinen beiden Flanken gewellte Nuten 25 zur Aufnahme einer elektrischen Leitung aufweisen, mit der die Elektrodeneinheiten 33 der Branchen 16, 17 mit Spannung oder Strom versorgt werden.

Die Branchen 16, 17 weisen jeweils einen Branchenträger 26 auf, wie er aus Figur 4 ersichtlich ist. Dieser kann aus Kunststoff oder Metall bestehen. Er ist als zweiarmiger Hebel ausgebildet und weist ein Werkzeugteil 27 sowie ein Betätigungsteil 28 auf. Zwischen beiden ist ein Lagerabschnitt 29 angeordnet, der in die Lagerbuchsen 22 oder 23 passt. Der Lagerabschnitt 29 ist im Wesentlichen zylindrisch ausgebildet. An einer Stirnfläche geht er nahtlos in die ebene Flanke des außermittig angeordneten Betätigungsteils 28 über. Außerdem ist der Lagerabschnitt 29 entlang eines streifenförmigen bzw. segmentförmigen Bereichs seines Zylindermantels mit dem Werkzeugteil 27 verbunden. Auf diese Weise ist eine sichere und belastbare schwenkbewegliche Lagerung des Branchenträgers 26 an dem Sockelteil 18 gegeben.

An dem Ende des Betätigungsteils 28 ist ein Betätigungszapfen 30 gehalten, der durch ein seitliches Fenster 31 in den Innenraum des Fortsatzes 20 ragt. Von dort aus kann ein an dem Betätigungszapfen 30 ansetzendes Zug- / Schubmittel längs durch den Schaft 12 in das Gehäuse 11 führen, um den Branchenträger 26 durch Betätigung des Bedienhebels 14 bewegen zu können.

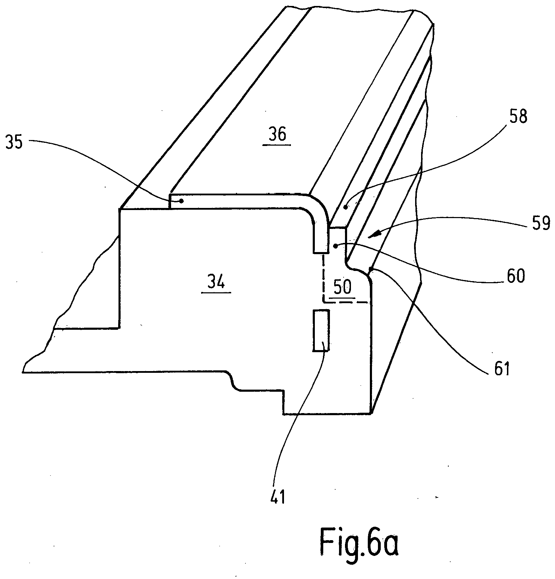

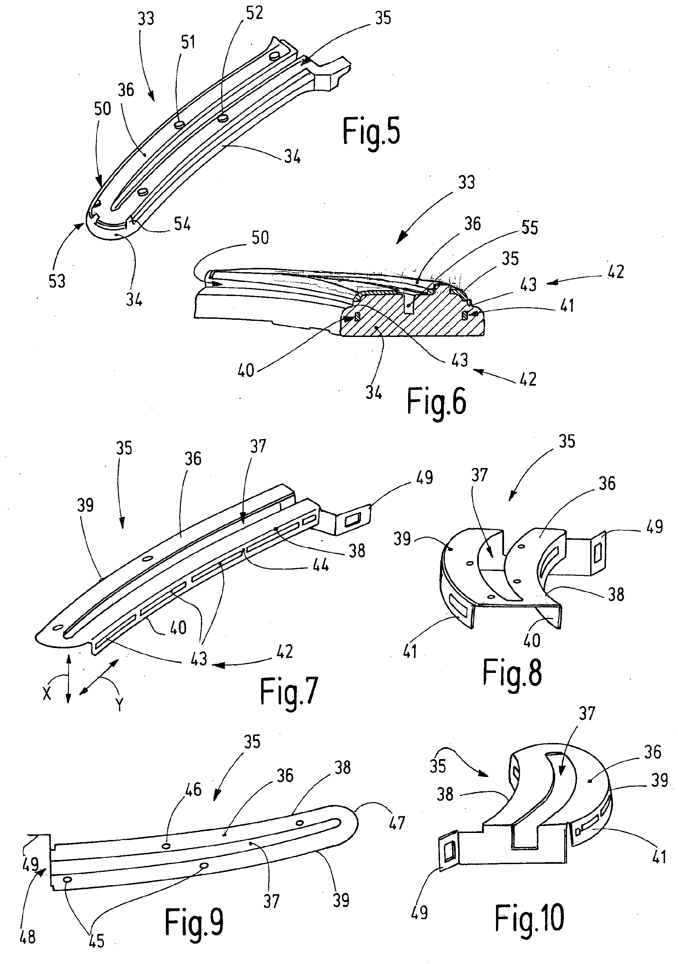

Der Werkzeugteil 27 weist eine Aufnahmefläche 32 zur Aufnahme einer Elektrodeneinheit 33 auf, die aus den Figuren 5 und 6 hervorgeht. Die Elektrodeneinheit 33 besteht aus einem Kunststoffkörper 34 und einem Blechteil 35. Das Blechteil 35 ist in den Figuren 7 bis 12 nochmals gesondert veranschaulicht.

Das Blechteil 35 bildet die Elektrode zur Einleitung von elektrischem Strom in ein biologisches Gewebe. Es weist eine vorzugsweise im Wesentlichen ebene Gewebekontaktfläche 36 auf, die mittig durch einen Längsschlitz 37 unterbrochen sein kann. Es erstrecken sich zu beiden Seiten des Längsschlitzes 37 länglich ausgeprägte ebene Abschnitte der Gewebekontaktfläche 36. Die Elektrodeneinheit 33 kann vollkommen gerade oder, wie aus den Figuren ersichtlich, leicht gekrümmt ausgebildet sein, um einer Krümmung des Werkzeugteils 27 zu folgen. Im letzteren Fall ist auch der Längsschlitz 37 entsprechend gekrümmt, so dass die länglichen Abschnitte der Gewebekontaktfläche 36 zu seinen beiden Seiten jeweils eine im Wesentlichen konstante Breite aufweisen, wie es aus Figur 9 hervorgeht.

Die Gewebekontaktfläche 36 weist zwei lange einander gegenüber liegende Ränder 38, 39 auf, von denen sich Streifenabschnitte 40, 41 abgewinkelt weg erstrecken. Die Ränder 38, 39 bilden dabei vorzugsweise gerundete Übergänge. Die Streifenabschnitte 40, 41 der Gewebekontaktfläche 36 sind zueinander vorzugsweise im Wesentlichen parallel angeordnet. Sie sind, weiter vorzugsweise, entlang ihrer gesamten Länge mit Öffnungen 42 versehen, die wie Figur 7 erkennen lässt, zum Beispiel als Schlitze 43 ausgebildet sein können. Diese Schlitze 43 sind durch Stege 44 voneinander getrennt, die vorzugsweise mindestens so lang wie breit sind. Die Länge wird dabei in einer Richtung senkrecht zu der Gewebekontaktfläche 36 verstanden. Diese Richtung ist in Figur 7 durch einen Pfeil X markiert. Die Breite wird parallel zu dem Längsschlitz 37 verstanden. Diese Richtung ist in Figur 7 durch einen Pfeil Y markiert. Ebenfalls in Längsrichtung, d.h. in Richtung des Pfeils Y weisen die Schlitze 43 eine Abmessung auf, die vorzugsweise wesentlich größer ist als ihre dazu senkrechte in X-Richtung gemessene Abmessung.

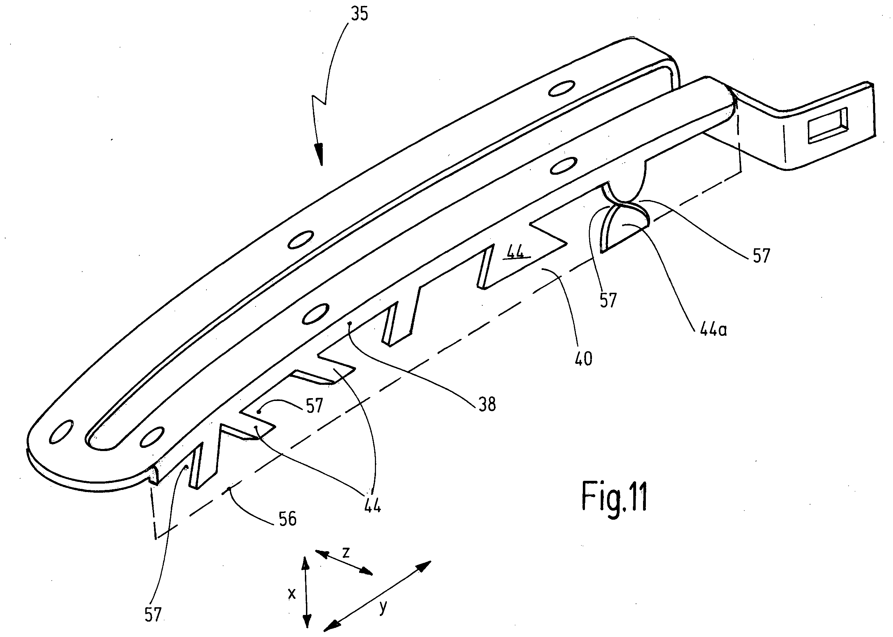

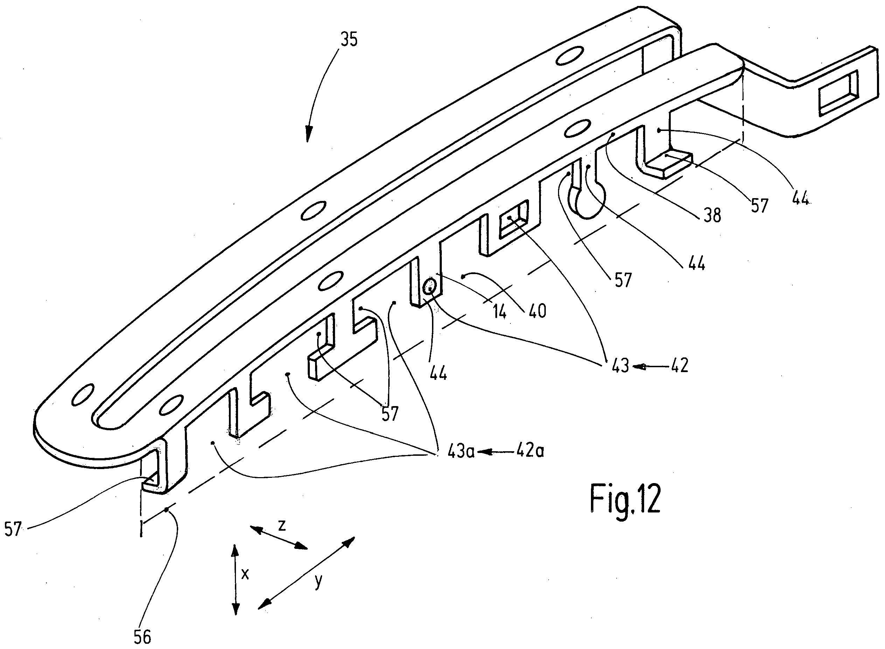

Wie aus den Ausführungsbeispielen der Figuren 11 und 12 ersichtlich ist, können die Streifenabschnitte 40, 41 verschiedene Formen aufweisen. Die Ausbildung der Streifenabschnitte ist beispielhaft an dem Streifenabschnitt 40 gezeigt und kann auch für den Streifenabschnitt 41 gelten. Der Streifenabschnitt 40 weist eine im Wesentlichen rechteckige Grundform auf, die sich von dem Rand 38 weg erstreckt. Diese Grundform ist in den Figuren 11 und 12 durch eine gestrichelte Linie 56 dargestellt. Der Streifenabschnitt 40 kann Schlitze 43 und Öffnungen 42 wie oben beschrieben und in den Figuren 7 und 8 dargestellt aufweisen. Zusätzlich oder alternativ kann der Streifenabschnitt 40 Öffnungen 42a, und Schlitze 43a, die eine von dem Rand 38 der Gewebekontaktfläche 36 weg erstreckende Form, in den Figuren 11 und 12 nach unten offene Form aufweisen. Die Schlitze 43a sind durch Stege 44 getrennt, deren obige Beschreibung entsprechend gilt. Die Öffnungen 42 und 42a sind so gestaltet, dass sie Haltemittel in Form von Ausnehmungen 57 oder Hinterschnitten 57 aufweisen, so dass der Kunststoffkörper 34 mit dem Blechteil 35 durch Formschluss verbunden werden kann. Um dies sicherzustellen, können die Stege 44 sich auch abschnittsweise in Z-Richtung erstrecken (Figur 12). Wie Figur 11 zeigt, ist es auch möglich, dass ein Steg 44a einen Bereich aufweist, der um seine eigene Achse verdreht ausgeführt ist. Darüber hinaus sind andere Ausführungsformen der Stege 44, 44a, der Öffnungen 42,42a und der Schlitze 43, 43a die dem Zwecke einer Formschlussbildung zwischen dem Kunststoffkörper 34 und dem Blechteil 35 dienen möglich.

Weiter sind in der Gewebekontaktfläche 36 Ausnehmungen 45, 46 ausgebildet, beispielsweise in Form von runden Löchern.

An dem distalen gerundeten Ende 47 weist das Blechteil 35 vorzugsweise keinen Streifenabschnitt auf. Bedarfsweise können jedoch auch hier von der Gewebekontaktfläche 36 über einen gerundeten Rand weg gebogene Abschnitte vorhanden sein. An dem gegenüber liegenden proximalen Ende 48 kann an einem abgewinkelten Abschnitt eine Anschlussfahne 49 ausgebildet sein, um stoff- oder formschlüssig beispielsweise durch Löten, Schweißen oder Crimpen eine Anschlussleitung zu befestigen.

Der Kunststoffkörper 34 füllt, wie Figur 6 zeigt, den Zwischenraum zwischen den Streifenabschnitten 40, 41 aus. Außerdem durchsetzt der Kunststoffkörper 34 die Schlitze 43, 43a oder sonstigen Öffnungen 42, 42a, die in den Streifenabschnitten 40, 41 ausgebildet sind. An den Außenseiten der Streifenabschnitte 40, 41 bildet der Kunststoffkörper 34 einen isolierenden Bereich, beispielsweise eine isolierende Stufe oder Schulter 50, die einen Abstand zwischen der Gewebekontaktfläche 36 und umgebendem, nicht der Behandlung zu unterziehendem Gewebe schafft. Die Schulter 50 weist einen im Wesentlichen rechteckigen Querschnitt mit einer an der Außenseite angeordneten Ausnehmung 59 auf. In Figur 6a ist der Querschnitt der Schulter 50 durch gestrichelte Linien angedeutet. In Richtung zur Gewebekontaktfläche 36 weist die Schulter 50 einen außen entlang der Gewebekontaktfläche 36 laufenden Vorsatz 60 auf, der mit einer gegenüber der Gewebekontaktfläche 36 tiefer gesetzten Fläche 58 abschließt. Durch diesen Vorsatz 60 wird die Gewebekontaktfläche 36 begrenzt und der Bereich des vom Strom durchflossenen, zwischen den Branchen gefassten Gewebes, festgelegt. Zusätzlich bildet dieser Vorsatz 60 einen Schutz für das Gewebe gegenüber der Gewebekontaktfläche 36 und verhindert bzw. reduziert in erheblichem Maße thermische Veränderungen des im Bereich der Schulter 50 liegenden Gewebes bei der Gewebeversiegelung. Die Ausnehmung 59 der Schulter 50 umfasst einen konkav gerundeten Abschnitt, so dass die Schulter einen konvex gekrümmten Bereich 61 aufweist. Durch den gerundeten konvex gekrümmten Bereich 61 wird beim Schließen der Branchen das Gewebe sicher gehalten, ohne es dabei zu verletzen. Damit dies sichergestellt ist, kann auch der Vorsatz 60 einen gerundeten Außenbereich aufweisen. Die Schulter 50 kann eine Breite und eine Höhe von 0,1 mm bis zu 3 mm, vorzugsweise 0,5 mm aufweisen. Damit kann die Koagulationswirkung sicher auf das zwischen den Branchen 16, 17 gefasste und zusammengedrückte Gewebe beschränkt werden. Das neben den Gewebekontaktflächen 36 zwischen den Schultern 50 gefasste Gewebe wird weniger oder nicht geklemmt. Die oben beschriebenen gerundet ausgebildeten Bereiche der Schulter 50 können auch andere Formen, beispielsweise abgewinkelte Bereiche umfassen. Die Schulter 50 ist gewebeschonend ausgebildet.

Der Kunststoffkörper 34 erstreckt sich außerdem durch die Ausnehmungen 45, 46 und bildet somit die Gewebekontaktfläche 36 überragenden Haltemittel 51, 52 (Figur 5), die dazu dienen, zwischen den Gewebekontaktflächen 36 gefasstes Gewebe auch dann sicher festzuhalten, wenn es infolge der Koagulation und Austrocknung schrumpft. Die Haltemittel 51, 52 können außerdem als Abstandshalter wirken, um eine metallische Berührung der Gewebekontaktflächen 36 der Elektrodeneinheiten 33 der beiden Branchen 16, 17 und somit einen elektrischen Kurzschluss verhindern.

Zentral in dem Längsschlitz 37 weist der Kunststoffkörper 34 eine Messerführungsnut 55 auf. Diese ist vorzugsweise deutlich schmaler als der Längsschlitz 37. Ein in der Messerführungsnut 55 laufendes Messer ist somit sicher gegen das Blechteil 35 elektrisch isoliert.

Zusätzlich zu den Haltemitteln 51, 52 können an der Elektrodeneinheit 33 Abstandshalter 53, 54 vorgesehen sein, die zum Beispiel durch von der Schulter 50 ausgehende Vorsprünge des Kunststoffkörpers 34 gebildet sind und die die Gewebekontaktfläche 36 überragen. Im Ausführungsbeispiel gemäß Figur 5 sind diese Abstandshalter 53, 54 am distalen Ende 47 der Elektrodeneinheit 33 angeordnet. Abstandshalter 53, 54 können bei anderen Ausführungsformen an anderen geeigneten Stellen der Elektrodeneinheit 33 angeordnet sein und können auch Mittel zum Halten des Gewebes bilden.

Das insoweit beschriebene Instrument 10 arbeitet wie folgt:
Das Instrument 10 wird mit seinem Anschlusskabel zunächst mit einem speisenden nicht weiter veranschaulichten Gerät bzw. Generator verbunden. Der Anwender kann dann biologisches Gewebe, beispielsweise Gefäße oder Gefäßbündel zwischen den Gewebekontaktflächen 36 der Branchen 16, 17 des Werkzeugs 15 fassen und durch Betätigung des Bedienhebels 14 festklemmen. Durch Betätigung eines nicht weiter veranschaulichten Schalters kann er nun eine elektrische Spannung, beispielsweise HF-Spannung, an die Gewebekontaktflächen 36 der Elektrodeneinheiten 33 der beiden Branchen 16, 17 anlegen, wodurch das geklemmte Gefäß elektrisch durchströmt wird. Dadurch kann eine Koagulation und Fusion einander gegenüber liegender Gefäßwände erreicht werden. Bei einigen Ausführungsformen ist außerdem ein Messer vorhanden. Dieses kann aktiviert werden, wobei es in der Messerführungsnut 55 in distaler Richtung vorgeschoben wird und dabei das zwischenzeitlich koagulierte und versiegelte Gefäß durchtrennt.

Während dieses Prozesses wird außerhalb der Branchen 16, 17 des Werkzeugs 15 liegendes biologisches Gewebe dabei von dem im Inneren ablaufenden elektrisch thermischen Prozess kaum oder gar nicht beeinflusst, da umliegendes Gewebe von den Gewebekontaktflächen 36 ferngehalten wird und die Schulter 50 des Kunststoffkörpers 34 wie auch der Kunststoffkörper 34 selbst sowie die Branchenträger 26 als elektrische und / oder thermische Isolatoren wirken.

Nach Gebrauch kann das Instrument 10 entsorgt oder recycelt werden. Es ist auch möglich, lediglich Teile oder das gesamte Instrument 10 zu sterilisieren.

Das erfindungsgemäße chirurgische Instrument 10 eignet sich insbesondere zur Gewebeversiegelung. Es umfasst ein Werkzeug 15, das wenigstens eine Branche 16 mit einer Elektrodeneinheit 33 aufweist. Diese besteht aus einem Blechteil 35, das vorzugsweise als Stanzbiegeteil ausgebildet und formschlüssig in einem vorzugsweise als Spritzgussteil ausgebildeten Kunststoffkörper 34 verankert ist. Die sich in den Kunststoffkörper 34 erstreckenden Teile, insbesondere Streifenabschnitte 40, 41, weisen großzügig dimensionierte Öffnungen 42 auf, durch die sich der Kunststoffkörper 34 erstreckt. So wird nicht nur eine formschlüssige Verankerung des Blechteils 35 in dem Kunststoffkörper 34, sondern zugleich auch eine Minimierung des Wärmeeintrags im Randbereich des Kunststoffkörpers 34 erreicht. Die Wärmekapazität des Blechteils 35 ist ebenso wie die Wärmeleitfähigkeit des Kunststoffkörpers 34 gering, so dass auch nach kurzfristig aufeinander folgendem mehrmaligem Gebrauch, ungeachtet der Anfangstemperatur der Gewebekontaktflächen 36, gleichbleibend gute Koagulationsergebnisse erreicht werden.

### Bezugszeichenliste:

- 10: Instrument
- 11: Gehäuse
- 12: Schaft
- 13: Handgriff
- 14: Bedienhebel
- 15: Werkzeug
- 16: erste Branche
- 17: zweite Branche
- 18: Sockelteil
- 19: Lagerabschnitt von 18
- 20: Fortsatz
- 21: Rastnase
- 22: Lagerbuchse für Branche 16
- 23: Lagerbuchse für Branche 17
- 24: Schlitz
- 25: Nut zur zugfesten Lagerung elektrischer Leitung
- 26: Branchenträger
- 27: Werkzeugteil
- 28: Betätigungsteil
- 29: Lagerabschnitt
- 30: Betätigungszapfen
- 31: Fenster
- 32: Aufnahmefläche
- 33: Elektrodeneinheit
- 34: Kunststoffkörper
- 35: Blechteil
- 36: Gewebekontaktfläche
- 37: Längsschlitz
- 38, 39: Ränder der Gewebekontaktfläche 36
- 40, 41: Streifenabschnitte
- 42, 42a: Öffnungen
- 43, 43a: Schlitze
- 44, 44a: Stege
- 45, 46: Ausnehmungen
- 47: distales Ende
- 48: proximales Ende
- 49: Anschlussfahne
- 50: Stufe, Schulter
- 51, 52: Haltemittel, Abstandsmittel
- 53, 54: distale Abstandshalter
- 55: Messerführungsnut
- 56: Rechteckige Grundform von 40, 41
- 57: Ausnehmung, Hinterschnitt
- 58: Fläche von 50
- 59: Ausnehmung von 50
- 60: Vorsatz von 50
- 61: gekrümmter Bereich von 50

## Patentansprüche

1. Chirurgisches Instrument (10), insbesondere zur Gewebefusion,
mit einem Werkzeug (15), das wenigstens eine Branche (16) mit einer Elektrodeneinheit (33) aufweist,
wobei die Elektrodeneinheit (33) aus einem Blechteil (35) und einem Kunststoffkörper (34) besteht und wobei:
- das Blechteil (35) eine Gewebekontaktfläche (36) mit zwei langen, einander gegenüberliegenden Rändern (38, 39) aufweist,
- sich von den Rändern (38, 39) abgewinkelte Streifenabschnitte (40, 41) weg erstrecken,
- in den Streifenabschnitten (40, 41) Öffnungen (42) vorgesehen sind,
- der Kunststoffkörper (34) einen zwischen den Streifenabschnitten (40, 41) ausgebildeten Zwischenraum ausfüllt
**dadurch gekennzeichnet,**
- **dass** der Kunststoffkörper (34) die Öffnungen (42, 42a) durchsetzt, indem er sich durch die Öffnungen (42, 42a) hindurch erstreckt,
- und **dass** der Kunststoffkörper (34) an den Außenseiten der Streifenabschnitte (40, 41) einen isolierenden Bereich (50) aufweist, der einen in Richtung zur Gewebekontaktfläche (36) außen entlang der Gewebekontaktfläche (36) verlaufenden Vorsatz (60) aufweist, der mit einer gegenüber der Gewebekontaktfläche (36) tiefer gesetzten Fläche (58) abschließt.

2. Instrument nach Anspruch 1, **dadurch gekennzeichnet, dass** die Öffnungen (42) Schlitze (43) sind.

3. Instrument nach Anspruch 2, **dadurch gekennzeichnet, dass** die Schlitze (43) durch Stege (44, 44a) voneinander getrennt sind, wobei die Fläche der Öffnungen (42, 42a) größer als die Fläche der Stege (44, 44a) ist.

4. Instrument nach Anspruch 3 **dadurch gekennzeichnet, dass** das Verhältnis der Flächen der Öffnungen (42, 42a) zu den Flächen der Stege (44, 44a) von 3 zu 1 bis 20 zu 1, vorzugsweise 10 zu 1 beträgt.

5. Instrument nach einem der vorstehenden Ansprüche, **dadurch gekennzeichnet, dass** die Gewebekontaktfläche (36) durch einen Längsschlitz (37) unterbrochen ist.

6. Instrument nach Anspruch 5, **dadurch gekennzeichnet, dass** der Kunststoffkörper (34) in fluchtender Anordnung zu dem Längsschlitz (37) eine Messerführungsnut (55) aufweist.

7. Instrument nach Anspruch 6, **dadurch gekennzeichnet, dass** der Längsschlitz (37) breiter ist als die Messerführungsnut (55).

8. Instrument nach einem der vorstehenden Ansprüche, **dadurch gekennzeichnet, dass** das Blechteil (35) ein durch Umformen oder Biegen gebildetes Teil ist.

9. Instrument nach einem der vorstehenden Ansprüche, **dadurch gekennzeichnet, dass** die Gewebekontaktfläche (36) Ausnehmungen (45, 46) aufweist, durch die sich der Kunststoffkörper (34) erstreckt, um Haltemittel (51, 52) zu bilden.

10. Instrument nach Anspruch 9, **dadurch gekennzeichnet, dass** die Haltemittel (51, 52) zu beiden Seiten eines Längsschlitzes (37) versetzt sind.

11. Instrument nach Anspruch 9, **dadurch gekennzeichnet, dass** ein Haltemittel (51, 52) eine flächenhafte Ausdehnung im Bereich von 0,5 mm² bis 7 mm² aufweist.

12. Instrument nach Anspruch 11, **dadurch gekennzeichnet, dass** ein Haltemittel (51, 52) eine Fläche von 0,75 mm² einnehmend ausgebildet sind.

13. Instrument nach einem der vorstehenden Ansprüche, **dadurch gekennzeichnet, dass** am Rand der Gewebekontaktfläche (36) Abstandshalter (53, 54) angeordnet sind.

## Claims

1. Surgical instrument (10), in particular for tissue fusion,
with a tool (15) which has at least one branch (16) with an electrode unit (33), wherein the electrode unit (33) consists of a sheet-metal part (35) and a plastic body (34), and wherein:
- the sheet-metal part (35) has a tissue contact face (36) with two long edges (38, 39) opposite each other,
- angled strip portions (40, 41) extend away from the edges (38, 39),
- openings (42) are provided in the strip portions (40, 41),
- the plastic body (34) fills a space formed between the strip portions (40, 41), **characterised in that**
- the plastic body (34) penetrates the openings (42, 42a) **in that** it extends through the openings (42, 42a),
- and the plastic body (34) has an insulating region (50) on the outsides of the strip portions (40, 41), which region has a protrusion (60) running in the direction towards the tissue contact face (36), on the outside along the tissue contact face (36), and terminating in a face (58) which is set lower than the tissue contact face (36).

2. Instrument according to claim 1, **characterised in that** the openings (42) are slots (43).

3. Instrument according to claim 2, **characterised in that** the slots (43) are separated from each other by webs (44, 44a), wherein the surface areas of the openings (42, 42a) are larger than the surface areas of the webs (44, 44a).

4. Instrument according to claim 3, **characterised in that** the ratio of the surface areas of the openings (42, 42a) to the surface areas of the webs (44, 44a) is from 3 : 1 to 20 : 1, preferably 10 : 1.

5. Instrument according to any of the preceding claims, **characterised in that** the tissue contact face (36) is interrupted by an elongate slot (37).

6. Instrument according to claim 5, **characterised in that** the plastic body (34) has a blade guide groove (55) arranged in alignment with the elongate slot (37).

7. Instrument according to claim 6, **characterised in that** the elongate slot (37) is wider than the blade guide groove (55).

8. Instrument according to any of the preceding claims, **characterised in that** sheet-metal part (35) is a part formed by forming or bending.

9. Instrument according to any of the preceding claims, **characterised in that** the tissue contact face (36) has recesses (45, 46) through which the plastic body (34) extends in order to form holding means (51, 52).

10. Instrument according to claim 9, **characterised in that** the holding means (51, 52) are offset to both sides of an elongate slot (37).

11. Instrument according to claim 9, **characterised in that** one holding means (51, 52) has a surface area in the range from 0.5 mm² to 7 mm².

12. Instrument according to claim 11, **characterised in that** a holding means (51, 52) is configured to have a surface area of 0.75 mm².

13. Instrument according to any of the preceding claims, **characterised in that** the spacers (53, 54) are arranged on the edge of the tissue contact face (36).

## Revendications

1. Instrument chirurgical (10), destiné notamment à la fusion tissulaire,
comprenant un outil (15) qui présente au moins un mors (16) avec une unité d'électrode (33),
dans lequel l'unité d'électrode (33) est constituée d'une pièce en tôle (35) et d'un corps en matière plastique (34), et dans lequel
- la pièce en tôle (35) présente une surface de contact de tissu (36) comportant deux bords (38, 39) longitudinaux situés en vis-à-vis l'un de l'autre,
- des portions de bande (40, 41) coudées s'étendent à partir des bords (38, 39),
- des ouvertures (42) sont prévues dans les portions de bande (40, 41),
- le corps en matière plastique (34) remplit un espace formé entre les portions de bande (40, 41),
**caractérisé en ce que**
- le corps en matière plastique (34) traverse les ouvertures (42, 42a) en passant à travers les ouvertures (42, 42a),
- et **en ce que** le corps en matière plastique (34) présente, sur les faces externes des portions de bande (40, 41), une zone isolante (50) comportant une partie saillante (60) qui s'étend à l'extérieur le long de la surface de contact de tissu (36), en direction de la surface de contact de tissu (36), et qui se termine par une surface (58) disposée plus bas par rapport à la surface de contact de tissu (36).

2. Instrument selon la revendication 1, **caractérisé en ce que** les ouvertures (42) sont des fentes (43).

3. Instrument selon la revendication 2, **caractérisé en ce que** les fentes (43) sont séparées les unes des autres par des barrettes (44, 44a), la superficie des ouvertures (42, 42a) étant plus grande que la superficie des barrettes (44, 44a).

4. Instrument selon la revendication 3, **caractérisé en ce que** le rapport entre les superficies des ouvertures (42, 42a) et les superficies des barrettes (44, 44a) va de 3 à 1 jusqu'à 20 à 1 et est de préférence de 10 à 1.

5. Instrument selon une des revendications précédentes, **caractérisé en ce que** la surface de contact de tissu (36) est interrompue par une fente longitudinale (37).

6. Instrument selon la revendication 5, **caractérisé en ce que** le corps en matière plastique (34) présente une rainure de guidage de lame (55), dans une disposition alignée avec la fente longitudinale (37).

7. Instrument selon la revendication 6, **caractérisé en ce que** la fente longitudinale (37) est plus large que la rainure de guidage de lame (55).

8. Instrument selon une des revendications précédentes, **caractérisé en ce que** la pièce en tôle (35) est une pièce façonnée par formage ou cintrage.

9. Instrument selon une des revendications précédentes, **caractérisé en ce que** la surface de contact de tissu (36) présente des évidements (45, 46) à travers lesquels s'étend le corps en matière plastique (34) pour former des moyens de maintien (51, 52).

10. Instrument selon la revendication 9, **caractérisé en ce que** les moyens de maintien (51, 52) sont décalés de part et d'autre d'une fente longitudinale (37).

11. Instrument selon la revendication 9, **caractérisé en ce qu'**un moyen de maintien (51, 52) présente une dimension de surface comprise dans la plage allant de 0,5 mm² à 7 mm².

12. Instrument selon la revendication 11, **caractérisé en ce qu'**un moyen de maintien (51, 52) est réalisé de façon à occuper une superficie de 0,75 mm².

13. Instrument selon une des revendications précédentes, **caractérisé en ce que** des éléments d'espacement (53, 54) sont disposés sur le bord de la surface de contact de tissu (36).
